# EUROPÄISCHE PATENTSCHRIFT

(11) **EP 1 618 873 B1**
(45) Veröffentlichungstag und Bekanntmachung des Hinweises auf die Patenterteilung: **06.06.2007**
(21) Anmeldenummer: 04016523.5
(22) Anmeldetag: 14.07.2004
(51) Int. Cl.: A61K 9/16, A61K 31/18

(54) **Granulat zur kontrollierten Freisetzung von Tamsulosin, enthaltend Alginat**
Granule for the controlled release of tamsulosin, containing alginate
Granule pour la libération contrôlée du tamsulosin, contenant de l'alginate

(43) Veröffentlichungstag der Anmeldung: 25.01.2006
(73) Patentinhaber: Siegfried Generics International AG, 4800 Zofingen (CH)
(72) Erfinder: Biro, Eszter Julianna, Dr., 5070 Frick (CH); Bueb, Waltraud, Dr., 4663 Aarburg (CH)
(74) Vertreter: Schaad, Balass, Menzl & Partner AG

(56) Entgegenhaltungen:
- WO-A-96/26717
- WO-A-99/39698
- WO-A-20/04050064
- WO-A-20/04056354
- DE-U- 20 219 293
- US-A- 4 772 475

## Beschreibung

Die Erfindung betrifft ein Granulat zur kontrollierten Freisetzung von Tamsulosin enthaltend eine Trägermatrix und Tamsulosin sowie ein Verfahren zur Herstellung des Granulates.

Tamsulosin ist der Trivialname von 5-[2-[[2-(2-ethoxyphenoxy)ethyl]amino]propyl]-2-methoxybenzolsulfonamid der Formel (I):

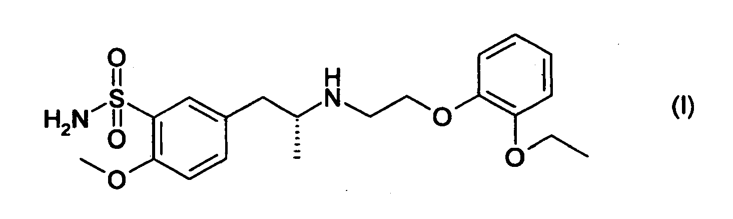

In EP 34432 und US 4731478 wird die pharmakologische Aktivität von Tamsulosin als alpha-adreneges Blockierungsmittel beschrieben, das wirksam bei der Behandlung von Herzbeschwerden und benigner Prostatahyperplasie ist.

Das Enantiomer R-Tamsulosin ist als Hydrochloridsalz in verschiedenen Ländern zur Behandlung der Symptome von benigner Prostatahyperplasie, wie beispielsweise von Blasenentleerungsstörungen, auf dem Markt. Das zugelassene Medikament enthält 0.4 mg Tamsulosin Hydrochlorid und wird oral als Kapsel verabreicht. Die Kapsel gibt das Tamsulosin im Körper kontrolliert ab und ist einmal täglich einzunehmen.

Es sind zahlreiche verschiedene Formulierungen enthaltend Tamsulosin bekannt.

US 4,772,475 (EP 194838, EP 533297) beschreibt die kontrollierte Freisetzung von Tamsulosin aus Kapseln, die eine mikrokristalline Cellulose und ein Agens für die kontrollierte Freisetzung des Tamsulosins enthalten.

In WO 03/039530 wird eine Tablette beschrieben, in der Tamsulosin trocken ohne Zugabe von Lösungsmitteln verpresst wird.

In WO 03/039531 wird eine Tablette beschrieben, die eine Matrix mit darin dispergiertem Tamsulosin enthält, wobei die Matrix wahlweise so beschichtet ist, dass weniger als 60% des Tamsulosins während 2 Stunden unter Bedingungen, die den Magen-Darm-Trakt simulieren, freigesetzt werden.

In WO 03/009831 wird eine schnell zerfallende Tablette aus Granulaten mit verzögerter Wirkstofffreisetzung beschrieben.

In EP 1043031 wird eine Gelformulierung enthaltend ionische Arzneistoffe, wie beispielsweise Tamsulosin, und entgegengesetzt geladene Hilfsstoffe beschrieben.

In WO 01/78725 wird ein Verfahren zur Herstellung kugelförmiger Pellets beschrieben. Dabei werden Lösungsmittel, Wirkstoff und mindestens ein Trägerstoff gemischt, wobei das Lösungsmittel nicht aufgesprüht wird. Die Mischung wird gerührt, das Lösungsmittel entfernt und die so erhaltenen Pellets getrocknet.

DE 202 19 293 U1 beschreibt ein Pellet mit einem Kern und einer Beschichtung, wobei der Kern Tamsulosin, mikrokristalline Cellulose, ein acrylisches Polymer und Wasser enthält. Die Beschichtung enthält ein säureresistentes acrylisches Polymer.

WO 2004/056354 beschreibt eine pharmazeutische Zusammensetzung mit kontrollierten Abgabeeigenschaften, die Tamsulosin enthält. Die Zusammensetzung ist ein Pellet mit einem Kern und eine diesen Kern umfassende Beschichtung.

US 4,772,475 beschreibt eine pharmazeutische Zusammensetzung mit kontrollierten Abgabeeigenschaften enthalten ein Granulationsprodukt, das erhalten wird, indem eine Abgabereagenz mit kontrollierenden Eigenschaften zu einer Mischung des physiologisch aktiven Wirkstoffs und einer Einheitsformenden Substanz gegeben wird, wobei die resultierende Mischung granuliert wird.

WO 2004/040064 (D4) beschreibt ein Pellet, das durch Lösen von Tamsulosin in einem hydrophilen Polymer hergestellt wurde, wobei die so entstandene Mischung mit einem Lösungsmittel versetzt wird, um eine Kombination mit einer ausreichenden Festigkeit zu bilden, so dass diese zu einem Faden extrudiert werden kann, unter anschliessendem Schmelzen dieser Fäden zu einem Pellet.

WO 96/26717 (D5) und WO 99/39698 (D6) beschreiben Zusammensetzungen mit kontrollierten Abgabeeigenschaften, das Alginat, ein Polymer, ein pH unabhängiges Polymer und einen Wirkstoff enthält.

Aufgabe der vorliegenden Erfindung ist es eine alternative Verabreichungsform sowie ein Verfahren zu deren Herstellung bereitzustellen, wobei die Verabreichungsform eine einheitliche Wirkstoffverteilung und ein kontrolliertes Freisetzungprofil aufweisen soll.

Die Aufgabe wird gelöst durch ein Granulat gemäss Anspruch 1 und das Verfahren gemäss Anspruch 12. Weitere bevorzugte Ausführungsformen sind Gegenstand der abhängigen Ansprüche.

Das Granulat enthält Tamsulosin und eine Trägermatrix. Die Trägermatrix enthält 2 bis 25 Gew.% eines Alginates, 30 bis 70 Gew.% einer makromolekularen Substanz und 10 bis 50 Gew.% einer hydrophoben Substanz. Die Kombination dieser Komponenten als Trägermatrix ermöglicht eine Zeit- und pH-abhängige Freisetzung von Tamsulosin und damit ein optimales Freisetzungsprofil in vivo. Durch die Wahl der Trägermatrix weist das erfindungsgemässe Granulat eine homogene Wirkstoffverteilung auf, selbst wenn der Wirkstoff in nur kleinen Mengen enthalten ist.

Das im erfindungsgemässen Granulat enthaltene Tamsulosin ist üblicherweise das R-Enantiomer von Tamsulosin, aber auch das S-Enantiomer oder eine Mischung der beiden in verschiedenen Verhältnissen wäre möglich. Tamsulosin kann als freie Base oder als Salz in dem Granulat enthalten sein. Mögliche Salze sind Tamsulosin-Hydrochlorid, Tamsulosinbesylat, Tamsulosinacetat, Tamsulosinmaleat, Tamsulosintartrat und Tamsulosincitrat. Das HydrochloridSalz ist bevorzugt in dem erfindungsgemässen Granulat enthalten.

Die Menge des in dem erfindungsgemässen Granulat enthaltenen Tamsulosins ist relativ tief, im allgemeinen tiefer als 10 mg, bevorzugt zwischen 0.1 und 1.2 mg und besonders bevorzugt zwischen 0.2 und 0.8 mg bezogen auf den eingesetzten Wirkstoff. In einer bevorzugten Ausführungsform enthält das erfindungsgemässe Granulat 0.4 mg Tamsulosin Hydrochlorid.

Das in der Trägermatrix des erfindungsgemässen Granulates enthaltene Alginat ist ausgewählt aus der Gruppe von Natriumalginat und Propylenglycolalginat. Natriumalginat ist eine Mischung von Polyuronsäuren bestehend aus D-Mannuronsäure und D-Guluronsäure. Das Verhältnis der D-Mannuronsäure und der D-Guluronsäure kann verschieden sein und beeinflusst die Gel-Eigenschaft der Trägermatrix sowie die Porosität des Gels. Bevorzugt beträgt der relative Anteil der D-Mannuronsäure 58 bis 62 Gew.% und der relative Anteil der D-Guluronsäure 38 bis 42 Gew.%. Im Magen wird das hydratisierte Natriumalginat in eine poröse, unlösliche Schicht umgewandelt. Im Darm, das heisst bei einem pH grösser 5.5, wird diese poröse, unlösliche Schicht zu einer löslichen viskosen Schicht. Enthält das erfindungsgemässe Granulat Propylenglycolalginat, weist es eine ausgesprochen gute Stabilität unter sauren Bedingungen auf. Bei einem höheren pH, das heisst im Darm, werden die Propylenglycol-Gruppen durch Hydrolyse abgespalten.

Die makromolekulare Substanz wird aus der Gruppe von Methacrylsäure-Ethylacrylat-Copolymer 1:1, Methacrylsäure-Methylmethacrylat Copolymere 1:1 oder 1:2, Aminoalkylmethacrylatcopolymere, Vinylacetat-Crotonsäurecopolymere, Polyvinylacetatphthalat, Ethylenvinylacetat, Celluloseacetatphthalat, Hydroxypropylmethylcellulose, Natriumcarboxymethylcellulose, Carrageenan, vernetztes Chitosan Polyethylenvinylacetat, Poly-L-Milchsäure, Xanthangummi und Polyvinylacetat oder Mischungen davon ausgewählt. Bevorzugt ist Methacrylsäure-Ethylacrylat-Copolymer in dem erfindungsgemässen Granulat enthalten, da dies dem Granulat eine gute mechanische Stärke verleiht. Methacrylsäure-Ethylacrylat-Copolymer ist bekannt unter dem Handelsnamen Eudragit L-100-55. Ebenfalls bevorzugt ist Polyvinylacetat, das zu einer sehr zusammenhängenden Trägermatrix führt und Polyvinylacetatphthalat, das zu einer sehr guten pH-abhängigen Freisetzung von Tamsulosin führt.

Die ebenfalls in dem erfindungsgemässen Granulat enthaltene hydrophobe Substanz wird ausgewählt aus der Gruppe von Glycerinbehenat, Glycerylmonostearat, Wachs, mono-, bis- und trisubstituierten Glyceriden und Calciumstearat oder Mischungen davon. Glycerinbehenat ist dabei bevorzugt. Glycerylbehenat ist eine Mischung von Glyceriden von Fettsäuren, hauptsächlich von Behensäure. Es gibt verschiedene Glycerinbehenate wie zum Beispiel 1,2,3-Propantriylester und Compritol^{®} 888 ato, das aus Mono-, Di- und Triglyceriden besteht, wobei der Anteil der Diglyceride überwiegt. Glycerinbehenat dient als Retardierungsmittel, sodass die Freisetzung des Tamsulosins aus dem erfindungsgemässen Granulat verzögert erfolgt.

Das erfindungsgemässe Granulat ist vorzugsweise in einer Hartgelatinekapsel enthalten, die sich im Magenbereich auflöst. Der Wirkstoff wird in kleinen Mengen im Magenbereich, aber insbesondere im Darmbereich freigesetzt.

In einer bevorzugten Ausführungsform enthält das erfindungsgemässe Granulat zusätzlich ein Bindemittel ausgewählt aus der Gruppe von Maltodextrin, Polyvinylpyrrolidon, pregelatinierter Stärke und Natriumstärkeglykolat. Besonders bevorzugt ist Maltodextrin, ein Malto-oligosaccharid, das durch eine Teilhydrolyse von Stärke hergestellt wird. Es ist ein natürliches Bindemittel, in kaltem Wasser lösbar und weist eine ausgezeichnete Benetzbarkeit auf. Das Bindemittel weist vorzugsweise eine Konzentration von 2 bis 25 Gew.%, besonders bevorzugt von 12 bis 18 Gew.% auf.

In einer weiteren Ausführungsform enthält das erfindungsgemässe Granulat zusätzlich ein oberflächenaktives Agens ausgewählt aus der Gruppe von Natriumlaurylsulfat, Poloxamer, Polyoxyethylenstearat und Polyoxyethylen-Rizinusöl-Derivaten, wie beispielsweise Polyoxyl 40 hydriertes Rizinusöl. Da Tamsulosin Hydrochlorid ein in Wasser nur schlecht löslicher Wirkstoff ist, kann zur Verbesserung der Löslichkeit ein oberflächenaktives Agens zugegeben werden. Besonders bevorzugt ist Natriumlaurylsulfat. Dieses anionische, oberflächenaktive Agens erhöht die Löslichkeit von Tamsulosin signifikant und kann die Freisetzung von Tamsulosin im Darm durch die mehr oder weniger zerstörte Struktur des Granulates begleiten. Das oberflächenaktive Agens weist vorzugsweise eine Konzentration von 0.5 bis 10 Gew.%, besonders bevorzugt von 1 bis 4 Gew.% auf.

In einer weiteren Ausführungsform enthält das erfindungsgemässe Granulat einen Weichmacher ausgewählt aus der Gruppe von Makrogol 6000, Polysorbat 80, Triethylcitrat, Acetyltriethylcitrat, Tributylcitrat, Propylenglycol, Diethylphthalat, Triacetin, Acetyltributylcitrat und Dibutylsebacat. Die Anwesenheit eines Weichmachers reduziert die Filmbildungstemperatur sowie die Glastemperatur des Methacrylsäure- Ethylacrylat-Copolymers beziehungsweise des Polyvinylacetats. Besonders bevorzugt sind dabei Makrogol 6000 und Polysorbat 80. Makrogol ist ein Kondensationspolymer von Ethylenoxid und Wasser. Die Zahl 6000 steht für das durchschnittliche Molekulargewicht. Polysorbat 80 entsteht bei der Veresterung von Sorbitol und seiner Anhydride mit Ölsäure und anschliessender Umsetzung mit Ethylenoxid. Jedes mol Sorbitol bzw. dessen Anhydrid weist ca. 20 mol Ethylenoxid auf. Polysorbat 80 hat zusätzlich einen dispersionsstabilisierenden Effekt. Der Weichmacher weist vorzugsweise eine Konzentration von 0.5 bis 5 Gew.%, besonders bevorzugt von 0.5 bis 2 Gew.% auf.

Dem erfindungsgemässen Granulat können weitere Hilfsstoffe wie ein pH adjustierendes Mittel, ein Antischaummittel und/oder ein Gleitmittel zugegeben werden.

Ein mögliches pH adjustierendes Mittel ist beispielsweise Natriumhydroxid. Der pH der Granulierflüssigkeit wird dabei vorzugsweise auf einen pH von ungefähr 5 eingestellt, indem kleine Mengen von Natriumhydroxid oder einer anderen dem Fachmann bekannten Base zugegeben werden. Dadurch werden teilweise die Carbonsäuregruppen der makromolekularen Substanz neutralisiert und dadurch deren Redispersibilisierbarkeit verbessert.

Die Zugabe eines Antischaummittels vereinfacht das Granulierungverfahren. Ein Beispiel dafür ist Simethicon, das die Oberflächenspannung reduziert. Bevorzugt wird das Antischaummittel in Mengen von 1 bis 100 ppm eingesetzt.

Durch die Anwesenheit eines Gleitmittels, wie beispielsweise kolloidales wasserfreies Silica, kann die Fliessbarkeit des Granulates erhöht werden. Das Gleitmittel weist vorzugsweise eine Konzentration von 0.1 bis 2 Gew.% auf.

Das erfindungsgemässe Granulat kann Flüssigkeiten enthalten wie Wasser und Lösungsmittel sowie Gemische davon. Mögliche Flüssigkeiten sind Ethanol, Ethanol/Wasser, Ethanol/Wasser, Isopropylalkohol, Isopropylalkohol/Wasser, Isopropylalkohol/Wasser, n-Butanol, Aceton, Aceton/Wasser, Aceton/Wasser, Aceton/Isopropylalkohol. Geeignete Mischungsverhältnisse sind dem Fachmann bekannt.

In einer besonders bevorzugten Ausführungsform enthält das erfindungsgemässe Granulat 0.15 bis 0.35 Gew.% Tamsulosin, 6 bis 8 Gew. % Natriumalginat, 55 bis 65 Gew. % Methacrylsäure-Ethylacrylat Copolymer 1:1, 12 bis 18 Gew.% Glycerinbehenat, 12 bis 18 Gew.% Maltodextrin und 0 bis 10 Gew.% Hilfsstoffe. Es wird vermutet, dass bei der Freisetzung von Tamsulosin dieses Granulat mit Flüssigkeit durchdrungen und hydratisiert wird. Dies hat ein Anschwellen des Alginates zur Folge, worauf das gelöste Tamsulosin durch die resultierende Gelschicht langsam diffundieren kann und die Alginatschicht im Darmtrakt abgebaut wird. Das hydrophobe Glycerylbehenat verlangsamt die Durchdringung von Flüssigkeit in die Gelschicht. Methacrylsäure- Ethylacrylat-Copolymer bildet teilweise einen Polymerfilm über und zwischen die Granulate, das die Freisetzung von Tamsulosin in saurer Umgebung verlangsamt. Durch die Anwesenheit des Methacrylsäure- Ethylacrylat-Copolymers erhält das erfindungsgemässe Granulat eine gute mechanische Stärke und kontrolliert die Diffusion des Tamsulosins bei seiner Freisetzung. Das Freisetzungsprofil dieses Granulates ist ausgezeichnet.

Das erfindungsgemässe Granulat wird hergestellt, in dem eine Standardgranulationstechnik mittels Schnellmischer (High-Shear-Mischer) verwendet wird. Dabei wird in zwei Teilschritten eine Pulvermischung und eine Granulierflüssigkeit hergestellt. Die Pulvermischung enthält das Alginat, einen Teil der makromolekularen Substanz, die hydrophobe Substanz und wahlweise ein Bindemittel. Diese Komponenten werden gemischt, bis eine homogene Pulvermischung erhalten wird.

Die Granulierflüssigkeit wird hergestellt, indem der restliche Teil der makromolekularen Substanz und das Tamsulosin zu dem Wasser oder dem Lösungsmittel gegeben werden. Die Flüssigkeit wird solange gerührt, bis eine homogene Lösung erhalten wird. In einer bevorzugten Ausführungsform wird das Wasser oder das Lösungsmittel erhitzt und in einem ersten Schritt ein Weichmacher zugegeben und gemischt. Die den Weichmacher enthaltende Lösung wird anschliessend abgekühlt. Durch die Anwesenheit des Weichmachers wird die Flexibilität der Granulierflüssigkeit und die Beschichtungseffizienz erhöht. In einem separaten Behälter wird wenigstens ein oberflächenaktives Agens, Tamsulosin und wahlweise ein Antischaummittel und/oder ein Gleitmittel gegeben und ebenfalls gemischt, bis eine homogene Lösung entsteht. Zu dieser Lösung wird der restliche Teil der makromolekularen Substanz zugegeben und weiter gerührt. Zu dieser Mischung wird schliesslich die den Weichmacher enthaltende Lösung langsam zugegeben und wahlweise noch ein pH adjustierendes Mittel dazugegeben, sodass eine homogene Granulierflüssigkeit entsteht.

Die homogene Granulierflüssigkeit wird unter starkem Rühren rasch zu der homogenen Pulvermischung zugegeben. Das starke Rühren ist wesentlich, um sicherstellen zu können, dass der Wirkstoff gut verteilt wird und die Partikelgrösse ungefähr homogen ist. Dies ist aufgrund der geringen Wirkstoffkonzentration des Tamsulosins wichtig, da so sichergestellt werden kann, dass jede Kapsel die gleiche Wirkstoffkonzentration aufweist. Alternativ kann die Granulierflüssigkeit aufgesprüht werden. Wenn die kleinen Partikel mit einer Granulierflüssigkeit mittlerer oder niedriger Viskosität gesprüht werden, wird das Tamsulosin optimal im Granulat verteilt, sodass die Wirkstoffkonzentration uniform ist.

Anschliessend wird das Granulat getrocknet. Dies kann beispielsweise in einem Trockenschrank mit oder ohne Vakuum, in einem Durchlufttrockenschrank, in einem Eintopfgranulator oder in einer Wirbelschichtanlage erfolgen. Das trockene Granulat wird anschliessend mit einer Hammermühle bearbeitet oder mittels oszillierendem oder rotierendem Sieb gesiebt, um so eine feine Partikelverteilung zu erhalten. Besonders gute Resultate und eine enge Partikelverteilung wurden mit der Hammermühle erhalten.

Die in den nachfolgenden Beispielen erwähnten Figuren zeigen:
- Figur 1:: eine Kurve der Freisetzung von Tamsulosin aus dem Granulat gemäss den Beispielen 1 bis 5,
- Figur 2:: eine Kurve der Freisetzung von Tamsulosin aus dem Granulat gemäss Beispiel 6,
- Figur 3:: eine Kurve der Freisetzung von Tamsulosin aus dem Granulat gemäss den Beispielen 6 bis 11,
- Figur 4:: eine Kurve der Freisetzung von Tamsulosin aus dem Granulat gemäss Beispiel 12,
- Figur 5:: eine Kurve der Freisetzung von Tamsulosin aus dem Granulat gemäss Beispiel 13,

### Beispiele

### Untersuchung der Freisetzung von Tamsulosin

### Testverfahren:

Der Freisetzungstest wurde mittels der Drehkörbchenmethode (rotating basket method) bei einer Rotationsgeschwindigkeit von 50 rpm durchgeführt. Bei dem Verfahren wurden die Bedingungen in dem Gastrointestinaltrakt simuliert, in dem die Medien nach einer gewissen Zeit verändert wurden, um den pH Gradienten zu simulieren. 450 ml des Mediums 1 wurde für eine Stunde belassen, anschliessend wurden 150 ml des Mediums 2 zugegeben.

| | |
|---|---|
| Medium 1: | 0.1 N HCl |
| Medium 2: | 0.05 M USP Puffer pH 6.8 |
| | 6.8 g KH₂PO₄ |
| | 22.4 ml 0.2 M NaOH |
| | mit Wasser auf 200 ml ergänzen |

### Apparatur und Analysebedingungen:

### Apparat Ph. Eur. (gegenwärtige Ausgabe (2004), Drehkörbchenapparatur)

Verfahren: Zuerst werden die Kapsel in 450 ml 0.1 N HCl für 1 Stunde gegeben. Dann werden 150 ml der Natriumphosphat-Pufferlösung zugegeben, die bei 37°C equilibriert worden war, um so eine Pufferlösung mit einem pH von 6.8 zu erhalten. Vor dem Beginn des Freisetzungstestes werden 450 ml 0.1 N HCl und 150 ml Natriumphosphatpuffer gemischt und der pH dieser Lösung gemessen. Sofern nötig, wurde der pH des Natriumphosphatpuffers angepasst, um einen pH der Lösung von 6.8 ± 0.05 zu erhalten.

| | |
|---|---|
| Volumen: | 450 ml (erste Stunde) -> 600 ml |
| Medium 1: | 100 ml 1 N Salzsäure wurde mit Wasser auf 1000 ml verdünnt. |
| Medium 2: | 450 ml HCl 0.1 N + 150 ml Natriumphosphatpuffer |
| Natriumphosphatpuffer: | 91 g Na₃PO₄ * 12 H₂O werden in Wasser gelöst und auf 1000 ml Wasser verdünnt. |
| Rührgeschwindigkeit: | 50 rpm |
| Temperatur: | 37°C ± 0.5 °C |
| Probenzeit: | Nach 0.5, 1, 2, 3, 4, 5 und 6 Stunden wurden ca. 2 ml jeder Testlösung entnommen und die Menge an gelöstem Tamsulosin mittels der unten angegebenen HPLC Methode bestimmt. |

### HPLC Methode zur Bestimmung von freigesetztem Tamsulosin

### Zubereitung der Referenzlösung

| | |
|---|---|
| Referenzstocklösung: | 20 bis 24 mg Tamsulosin HCl Referenzsubstanz wurden exakt abgewogen und in einen 100 ml Messzylinder gegeben und mit 0.1 N Salzsäure auf das entsprechende Volumen verdünnt. Der Messzylinder wurde bei Raumtemperatur in ein Ultraschallbad gestellt bis Tamsulosin HCl vollständig gelöst war. |
| Referenzlösung 1: | 10.0 ml der Referenzstocklösung wurde in einen 200 ml Messzylinder gegeben und auf das Volumen mit 0.1 N Salzsäure verdünnt. 20.0 ml der so erhaltenen Lösung wurde in einen 250 ml Messzylinder gegeben und auf das Volumen mit 0.1 N Salzsäure verdünnt. (Konzentration Tamsulosin HCl 0.88 µg/ml). |
| Referenzlösung 2: | 10.0 ml der Referenzstocklösung wurde in einen 200 ml Messzylinder gegeben und auf das Volumen mit dem Medium 2 verdünnt. 15.0 ml der so erhaltenen Lösung wurde in einen 250 ml Messzylinder gegeben und auf das Volumen mit dem Medium 2 verdünnt. (Konzentration Tamsulosin HCl 0.66 µg/ml). |

### Eignungstest des Systems für die Freisetzungsanalyse

Der Eignungstest für das System wurde vor dem HPLC Freisetzungstest der 0.4 mg Tamsulosinkapseln durchgeführt.

Verfahren: Die Referenzlösung 1 wird 5 mal eingespritzt und die Retentionszeit in Form einer Bande aufgezeichnet. Die berechnete relative Standard - Abweichung (RSD) der Tamsulosin-Banden wird berechnet. Das Ende der Bande (tailing) wird bei der Bande der 5. Einspritzung bestimmt.

| | | |
|---|---|---|
| Spezifizierung: | RSD | ≤ 2.0% |
| | Ende der Bande | ≤ 1.5 |
| HPLC Bedingungen: Kolonne: | Nucleosil 100-5 C-18 AB, 125 x 3 mm, Partikelgrösse 5 µm. | |
| Lösungsmittel: | 780 ml Puffer, 220 ml Acetonitril | |
| Puffer: | H₃PO₄ wird zu 0.05M KH₂PO₄ gegeben bis der pH 3.0 ± 0.05 ist. | |
| Dauer der Analyse: | 5 Minuten | |
| Einspritzvolumen: | 50 µl | |
| Kolonnentemperatur: | 40°C | |
| Fluss: | 1.0 ml/min | |
| UV-Detektion: | Signal: 225.4 nm, | |
| | Referenz: 550, 100 nm | |

### Beispiele 1 bis 5

Das Granulat wurde hergestellt, indem eine StandardGranulationstechnik mit einem High-Shear Granulator verwendet wurde.

Die Pulvermischung enthielt eine Trägermatrix enthaltend Natriumalginat, Glycerolbehenat und Polyvinylacetatphthalat und das Bindemittel Maltodextrin.

Die Granulierflüssigkeit war eine wässrige Dispersion enthaltend Polyvinylacetatphthalat, gelöstes Tamsulosin und das oberflächenaktives Agens Natriumlaurylsulfat und Polyoxamer, wobei auch andere als Löslichkeitsvermittler dienende oberflächenaktive Agenzien hätten eingesetzt werden können.

Während des feuchten Zusammenknetens bildete die Granulierflüssigkeit durch Agglomeration der Pulverpartikel Granulate. Nach dem Trocknen der Granulate wurden diese mit einem Oszillationssieb gesiebt. Die getrockneten Granulate wurden anschliessend in die Hartgelatinekapseln gefüllt.

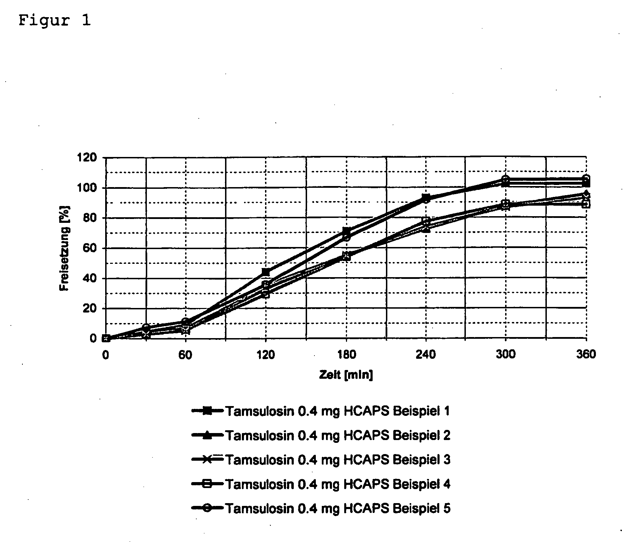

Die Freisetzung des Tamsulosins kann innerhalb eines breiten Bereichs kontrolliert werden (siehe Figur 1), indem die Mengen der einzelnen Komponenten in der Trägermatrix verändert wird. Daher wurden die Granulate mit untersucht, bei der die Menge der einzelnen Komponenten variiert wurde. Tabelle 1 zeigt die Zusammensetzung der Granulate.

**Tabelle 1: Beispiele 1-5**

| **Beispiel** | **1** | **2** | **3** | **4** | **5** |
|---|---|---|---|---|---|
| Zusammensetzung: | w/w % | w/w % | w/w % | w/w % | w/w % |
| Tamsulosin HCl* | 0.18 | 0.18 | 0.18 | 0.18 | 0.18 |
| Natriumlaurylsulfat* | 1.00 | 1.00 | 1.00 | 1.00 | 1.00 |
| Poloxamer 407* | 0.25 | 0.25 | 0.25 | 0.25 | 0.25 |
| Simethicon* | 0.01 | 0.01 | 0.04 | 0.04 | 0.04 |
| Maltodextrin | 10.00 | 10.00 | 10.00 | 12.00 | 28.53 |
| Natriumalginat | 10.00 | 20.00 | 7.50 | 12.00 | 10.00 |
| Glyceroldibehenat | 20.00 | 10.00 | 17.50 | 18.53 | 0.00 |
| Polyvinylacetatphthalat** | 58.56 | 58.56 | 63.53 | 56.00 | 60.00 |

| | | | | | |
|---|---|---|---|---|---|
| * in der Granulierflüssigkeit ** teilweise in der Granulierflüssigkeit | | | | | |

### Beispiel 6

Es wurde das gleiche Verfahren wie bei den Beispielen 1 bis 5 angewendet. Dabei wurde die in Tabelle 2, Spalte 2, angegebene Zusammensetzung verwendet. Polyvinylacetatphthalat wurde durch Methacrylsäure-Ethylacrylat Copolymers ersetzt (1:1). Durch die Zugabe des Weichmachers erhielt die Dispersion des Methacrylsäure-Ethylacrylat Copolymer (1:1) die notwendige Flexibilität. Macrogol 6000 wurde als Weichmacher ausgewählt. Das oberflächenaktive Agens Poloxamer 407 wurde durch Polysorbat 80 wegen dessen dispersionsstabilisierenden Effektes ersetzt. Das heisst, dass die Homogenität der Granulierflüssigkeit besser wird.

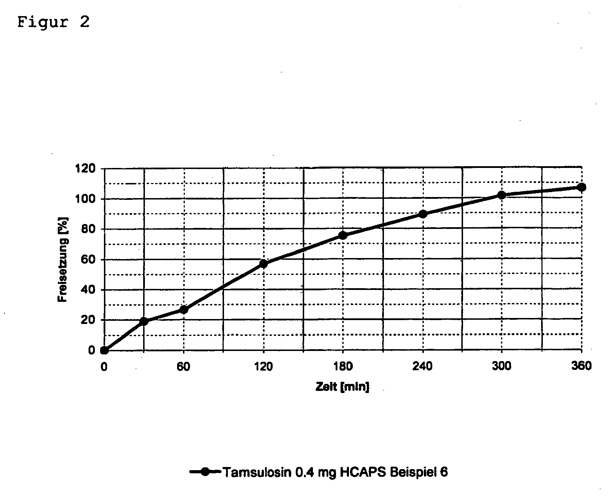

Zusätzlich wurde eine Natriumhydroxidlösung verwendet, um den pH der Polymerdispersion anzupassen. Das Freisetzungsprofil ist in Figur 2 abgebildet.

### Beispiel 7 bis 11

Es wurde das gleiche Verfahren wie in Beispiel 6 beschrieben durchgeführt unter Verwendung der in Tabelle 2 unten gezeigten Granulat-Zusammensetzungen. Die Reihenfolge der Zugabe der Inhaltsstoffe und die Zusammensetzung der Granulierflüssigkeit wurden verändert, ohne dass in der quantitativen Zusammensetzung des Granulats etwas wesentliches verändert wurde.

### Beispiel 7: Die Eudragitmenge wurde auf 15% in der Granulierflüssigkeit reduziert (vorher 20%)

### Beispiel 8: Die Granulierflüssigkeit wurde ohne Eudragit, Polysorbat und Natriumhydroxid hergestellt.

### Beispiel 9: Das Natriumalginatpulver wurde nach dem Befeuchten der Pulvermischung zugegeben.

### Beispiel 10: Weniger Wasser in der Granulierflüssigkeit

### Beispiel 11: Granulierflüssigkeit wurde ohne Eudragit, Polysorbat, Natriumhydroxid und Macrogol zubereitet.

**Tabelle 2:**

| **Beispiel w/w %** | **6** | **7** | **8** | **9** | **10** | **11** |
|---|---|---|---|---|---|---|
| Tamsulosin HCl | * 0.21 | * 0.24 | * 0.24 | * 0.24 | * 0.25 | * 0.25 |
| Natriumalginat | 7.00 | 7.00 | 7.00 | 7.00 | 7.00 | 7.14 |
| Methacrylsäure - Ethylacrylat-Copolymer 1:1 | (9.67 + 49.36) # 59.03 | # 60.37 | 59.81 | # 58.92 | 59.82 | 61.00 |
| Glyceroldibehenat | 15.00 | 15.00 | 15.00 | 15.00 | 15.00 | 15.29 |
| Maltodextrin | 15.00 | 15.00 | 15.00 | 15.00 | 15.00 | 15.29 |
| Natriumlaurylsulfat | * 1.00 | * 1.00 | * 1.00 | * 1.00 | * 1.00 | * 1.02 |
| Macrogol 6000 | * 1.94 | * 0.97 | * 1.94 | * 2.00 | * 1.92 | 0.00 |
| Polysorbat 80 | * 0.76 | * 0.39 | 0.00 | * 0.80 | 0.00 | 0.00 |
| Natriumhydroxid | * 0.05 | * 0.02 | 0.00 | * 0.03 | 0.00 | 0.00 |
| Simethicon | * 0.01 | * 0.01 | * 0.01 | * 0.01 | * 0.01 | * 0.01 |

| | | | | | | |
|---|---|---|---|---|---|---|
| * in der Granulierflüssigkeit # teilweise in der Granulierflüssigkeit | | | | | | |

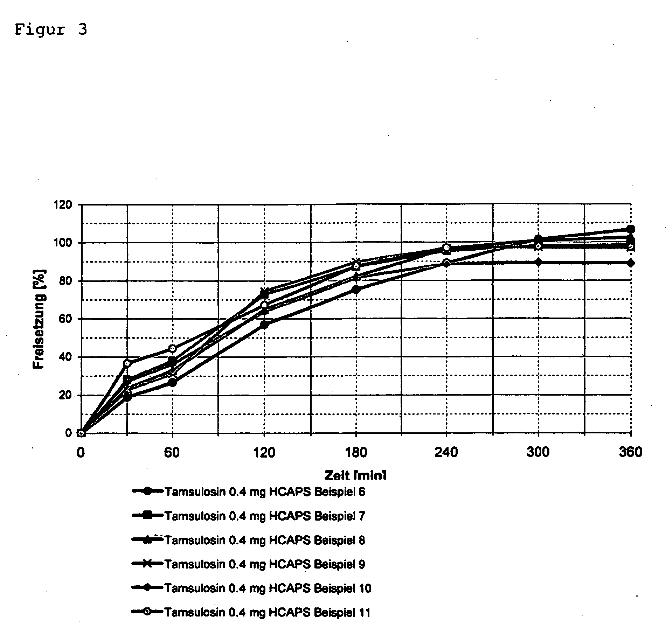

Wie in Figur 3 gezeigt, führt eine kleinere Menge von Eudragit und/oder Wasser zu weniger Filmregionen in dem Granulat, was zu einer schnelleren Freisetzung im Vergleich mit dem Freisetzungsprofil des Beispiels 6 führt.

### Beispiel 12:

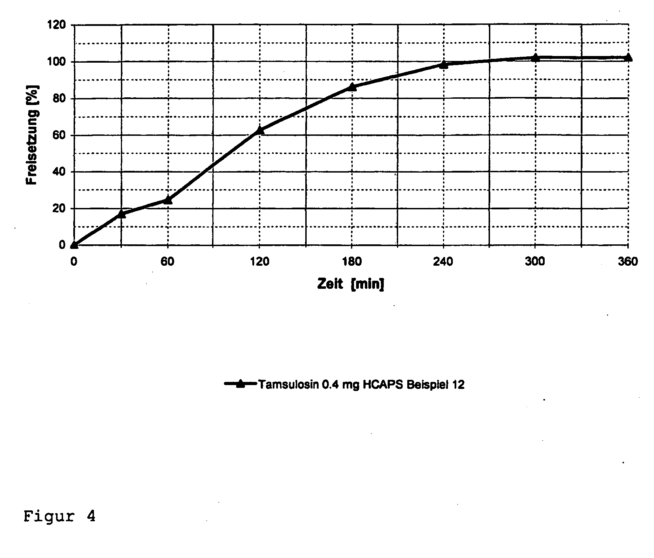

Indem das gleiche Herstellungsverfahren wie in Beispiel 6 angewendet wird unter Verwendung der in Tabelle 3 gezeigte Zusammensetzung, wurden Tamsulosin enthaltendes Granulat hergestellt (siehe Figur 4).

Die Verteilung des Tamsulosin wurde durch die Veränderung der Siebschritte nach der Trocknung verbessert. Das Oszillationssieb wurde durch eine Hammermühle ersetzt. Um die Fliessbarkeit des Granulates zu erhöhen, wurde kolloidales wasserfreies Silica als Gleitmittel zugegeben und mit den getrockneten und gesiebten Granulaten gemischt.

**Tabelle 3:**

| **Beispiel** | **12** |
|---|---|
| **w/w %** | |
| Tamsulosin HCl | * 0.31 |
| Natriumalginat | 7.00 |
| Methacrylsäure-Ethylacrylat Copolymer 1:1 | # 58.94 |
| Glyceroldibehenat | 15.00 |
| Maltodextrin | 15.00 |
| Natriumlaurylsulfate | * 1.15 |
| Macrogol 6000 | * 1.69 |
| Polysorbat 80 | * 0.65 |
| Natriumhydroxid | * 0.05 |
| Simethicon | * 0.01 |
| Kolloidales wasserfreies Silika | 0.20 |

| | |
|---|---|
| * in der Granulierflüssigkeit # *teilweise* in *der Granulierflüssigkeit* | |

### Beispiel 13

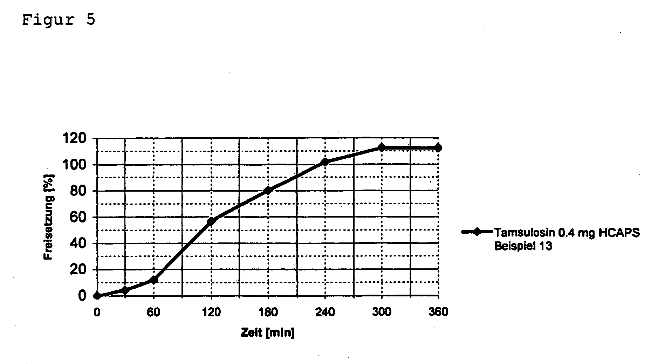

Indem das gleiche Verfahren wie bei Beispiel 6 unter Verwendung der Zusammensetzung gemäss Tabelle 4 angewendet wurde, wurden Tamsulosin Granulate hergestellt und zusätzlich mit einem Polymerfilm von Methacrylsäure-Ethylacrylat Copolymer 1:1 mittels Wirbelschicht-Verfahren hergestellt. Das Freisetzungsprofil ist in Figur 5 abgebildet.

**Tabelle 4**

| **Zusammensetzung** | w/w % |
|---|---|
| **Matrixgranulat** | |
| **Granulierflüssigkeit** | |
| Tamsulosin HCl | 0.17 |
| Methacrylsäure - Ethylacrylat Copolymer 1:1 | 13.25 |
| Macrogol 6000 | 1.32 |
| Polysorbate 80 | 0.53 |
| Natriumlaurylsulfat | 0.63 |
| Natriumhydroxid | 0.07 |
| Simethicon | 0.01 |

| **Pulvermischung** | |
|---|---|
| Mikrokristalline Cellulose | 20.00 |
| Calciumstearat | 4.00 |
| Vorgelatinisiertes acetyliertes Distärkeadipat | 38.02 |

| **Filmbeschichtung** | |
|---|---|
| Methacrylsäure - Ethylacrylat Copolymer 1:1 | 19.39 |
| Macrogol 6000 | 1.89 |
| Glycerolmonostearat | 0.49 |
| Polysorbat 80 | 0.21 |
| Simethicon | 0.02 |
| Total | 100 |

## Patentansprüche

1. Granulat zur kontrollierten Freisetzung von Tamsulosin enthaltend Tamsulosin und eine Trägermatrix, wobei die Trägermatrix
a) 2 bis 25 Gew.% eines Alginates,
b) 30 bis 70 Gew.% einer makromolekularen Substanz ausgewählt aus der Gruppe von Methacrylsäure-Ethylacrylat-Copolymer 1:1, Methacrylsäure-Methylmethacrylat-Copolymer 1:1 oder 1:2, Aminoalkylmethacrylatcopolymer, Vinylacetat-Crotonsäure-Copolymer, Polyvinylacetatphthalat, Ethylenvinylacetat, Celluloseacetatphthalat, Hydroxypropylmethylcellulose, Natriumcarboxymethylcellulose, Carrageenan, vernetztes Chitosan Polyethylenvinylacetat, Poly-L-Milchsäure, Xanthangummi und Polyvinylacetat oder Mischungen davon, und
c) 10 bis 50 Gew.% einer hydrophoben Substanz ausgewählt aus der Gruppe von Glycerinbehenat, Glycerylmonostearat, Wachs, mono-, bis- und trisubstituierten Glyceriden und Calciumstearat oder Mischungen davon
enthält.

2. Granulat gemäss Anspruch 1, wobei die makromolekulare Substanz Methacrylsäure-Ethylacrylat-Copolymer ist.

3. Granulat gemäss einem der vorangehenden Ansprüche, wobei die hydrophobe Substanz Glycerinbehenat ist.

4. Granulat gemäss einem der vorangehenden Ansprüche, enthaltend ein Bindemittel ausgewählt aus der Gruppe von Maltodextrin, Polyvinylpyrrolidon, pregelatinierte Stärke und Natriumstärkeglycolat oder Mischungen davon.

5. Granulat gemäss einem der vorangehenden Ansprüche, enthaltend ein oberflächenaktives Agens ausgewählt aus der Gruppe von Natriumlaurylsulfat, Poloxamer, Polyoxyethylenstearat und Polyoxyethylen-Rizinusöl-Derivaten oder Mischungen davon.

6. Granulat gemäss einem der vorangehenden Ansprüche, enthaltend einen Weichmacher ausgewählt aus der Gruppe von Kondensationspolymeren von Ethylenoxid und Wasser, mit Ethylenoxid umgesetzten Sorbitolölsäureester und deren Anhydride, Triethylcitrat, Acetyltriethylcitrat, Tributylcitrat, Propylenglycol, Diethylphthalate, Triacetin, Acetyltributylcitrat und Dibutylsebacat oder Mischungen davon.

7. Granulat gemäss einem der vorangehenden Ansprüche, enthaltend ein pH adustierendes Mittel, ein Antischaummittel und/oder ein Gleitmittel.

8. Granulat gemäss einem der vorangehenden Ansprüche, enthaltend Wasser oder ein Lösungsmittel oder Mischungen davon, wobei das Lösungsmittel ausgewählt ist aus der Gruppe von Ethanol, Isopropylalkohol und Aceton.

9. Granulat gemäss einem der vorangehenden Ansprüche enthaltend
| | |
|---|---|
| 0.15 bis 0.35 Gew.% | Tamsulosin |
| 6 bis 8 Gew. % | Natriumalginat |
| 55 bis 65 Gew. % | Methacrylsäure-Ethylacrylat Copolymer 1:1 |
| 12 bis 18 Gew.% | Glycerinbehenat |
| 12 bis 18 Gew.% | Maltodextrin |
| 0 bis 10 Gew.% | Hilfsstoffe. |

10. Hartgelatinekapsel enthaltend das Granulat nach einem der vorangehenden Ansprüche.

11. Verfahren zur Herstellung eines Granulates gemäss einem der Ansprüche 1 bis 9 enthaltend Tamsulosin, in dem
das Natriumalginat, ein erster Teil der makromolekularen Substanz und die hydrophobe Substanz gemischt werden, wobei ein homogenes Pulvergemisch erhalten wird,
Wasser oder ein Lösungsmittel, ein zweiter Teil der makromolekularen Substanz und Tamsulosin gemischt werden, wobei eine homogene Granulierflüssigkeit erhalten wird, und
die Granulierflüssigkeit unter starkem Rühren zu dem homogenen Pulvergemisch gegeben und das erhaltene Granulat getrocknet wird.

12. Verfahren gemäss Anspruch 11, wobei die homogene Pulvergemisch erhalten wird, indem Natriumalginat, ein erster Teil der makromolekularen Substanz, die hydrophobe Substanz und ein Bindemittel gemischt werden.

13. Verfahren gemäss Anspruch 11, wobei die Granulierflüssigkeit erhalten wird, indem
Wasser oder das Lösungsmittel erhitzt, dazu ein Weichmacher gegeben und gemischt wird und die so erhaltene, den Weichmacher enthaltende Lösung abgekühlt wird,
Tamsulosin und wenigstens ein oberflächenaktives Agens gemischt werden und zu dieser Mischung der zweite Teil der makromolekularen Substanz zugegeben und gemischt wird, und
die so erhaltene Mischung zu der den Weichmacher enthaltenden Lösung gegeben wird.

## Claims

1. Granules for controlled release of tamsulosin comprising tamsulosin and a carrier matrix, where the carrier matrix comprises
a) 2 to 25% by weight of an alginate,
b) 30 to 70% by weight of a macromolecular substance selected from the group of methacrylic acid/ethyl acrylate 1:1 copolymer, methacrylic acid/methyl methacrylate 1:1 or 1:2 copolymers, aminoalkyl methacrylate copolymer, vinyl acetate/crotonic acid copolymer, polyvinyl acetate phthalate, ethylene-vinyl acetate, cellulose acetate phthalate, hydroxypropylmethylcellulose, sodium carboxymethylcellulose, carrageenan, crosslinked chitosan, polyethylene-vinyl acetate, poly-L-lactic acid, xanthan gum and polyvinyl acetate or mixtures thereof, and
c) 10 to 50% by weight of a hydrophobic substance selected from the group of glycerol behenate, glyceryl monostearate, wax, mono-, di- and trisubstituted glycerides and calcium stearate or mixtures thereof.

2. Granules as claimed in claim 1, where the macromolecular substance is methacrylic acid/ethyl acrylate copolymer.

3. Granules as claimed in either of the preceding claims, where the hydrophobic substance is glycerol behenate.

4. Granules as claimed in any of the preceding claims, comprising a binder selected from the group of maltodextrin, polyvinylpyrrolidone, pregelatinized starch and sodium starch glycolate or mixtures thereof.

5. Granules as claimed in any of the preceding claims, comprising a surface-active agent selected from the group of sodium lauryl sulfate, poloxamer, polyoxyethylene stearate and polyoxyethylene castor oil derivatives or mixtures thereof.

6. Granules as claimed in any of the preceding claims, comprising a plasticizer selected from the group of condensation polymers of ethylene oxide and water, sorbitol oleic acid ester reacted with ethylene oxide, and anhydrides thereof, triethyl citrate, acetyl triethyl citrate, tributyl citrate, propylene glycol, diethyl phthalate, triacetin, acetyl tributyl citrate and dibutyl sebacate or mixtures thereof.

7. Granules as claimed in any of the preceding claims, comprising a pH-adjusting agent, an antifoam and/or a glidant.

8. Granules as claimed in any of the preceding claims, comprising water or a solvent or mixtures thereof, where the solvent is selected from the group of ethanol, isopropyl alcohol and acetone.

9. Granules as claimed in any of the preceding claims, comprising
| | |
|---|---|
| 0.15 to 0.35% | by weight of tamsulosin |
| 6 to 8% | by weight of sodium alginate |
| 55 to 65% | by weight of methacrylic acid/ethyl acrylate 1:1 copolymer |
| 12 to 18% | by weight of glycerol behenate |
| 12 to 18% | by weight of maltodextrin |
| 0 to 10% | by weight of excipients. |

10. A hard gelatin capsule comprising the granules as claimed in any of the preceding claims.

11. A process for producing granules as claimed in any of claims 1 to 9 comprising tamsulosin, in which
the sodium alginate, a first part of the macromolecular substance and the hydrophobic substance are mixed, resulting in a homogeneous powder mixture,
water or a. solvent, a second part of the macromolecular substance and tamsulosin are mixed, resulting in a homogeneous granulating liquid, and
the granulating liquid is added with vigorous stirring to the homogeneous powder mixture, and the resulting granules are dried.

12. The process as claimed in claim 11, where the homogeneous powder mixture is obtained by mixing sodium alginate, a first part of the macromolecular substance, the hydrophobic substance and a binder.

13. The process as claimed in claim 11, where the granulating liquid is obtained by
heating water or the solvent, adding a plasticizer thereto and mixing, and cooling the solution obtained in this way and comprising the plasticizer,
mixing tamsulosin and at least one surface-active agent, and adding to this mixture the second part of the macromolecular substance, and mixing, and
adding the mixture obtained in this way to the solution comprising the plasticizer.

## Revendications

1. Granulat pour la libération contrôlée de Tamsulosine contenant de la Tamsulosine et une matrice support, la matrice support contenant
a) 2 à 25% en poids d'un alginate,
b) 30 à 70% en poids d'une substance macromoléculaire choisie dans le groupe formé par le copolymère d'acide méthacrylique-acrylate d'éthyle 1:1, le copolymère d'acide méthacrylique-méthacrylate de méthyle 1:1 ou 1:2, le copolymère de méthacrylate d'aminoalkyle, le copolymère d'acétate de vinyle-acide crotonique, le poly(acétate-phtalate de vinyle), l'éthylène-acétate de vinyle, l'acétate-phtalate de cellulose, l'hydroxypropylméthylcellulose, la carboxyméthylcellulose de sodium, le carraghénane, le chitosane réticulé, le poly(éthylène-acétate de vinyle), le poly(acide L-lactique), la gomme de xanthane et le poly(acétate de vinyle) ou les mélanges de ceux-ci, et
c) 10 à 50% en poids d'une substance hydrophobe choisie dans le groupe formé par le béhénate de glycérol, le monostéarate de glycéryle, la cire, les glycérides monosubstitués, disubstitués et trisubstitués et le stéarate de calcium ou les mélanges de ceux-ci.

2. Granulat selon la revendication 1, la substance macromoléculaire étant le copolymère d'acide méthacrylique-acrylate d'éthyle.

3. Granulat selon l'une quelconque des revendications précédentes, la substance hydrophobe étant le béhénate de glycérol.

4. Granulat selon l'une quelconque des revendications précédentes, contenant un liant choisi dans le groupe formé par la maltodextrine, la polyvinylpyrrolidone, l'amidon prégélatinisé et le glycolate sodique d'amidon ou des mélanges de ceux-ci.

5. Granulat selon l'une quelconque des revendications précédentes, contenant un agent tensioactif choisi dans le groupe formé par le laurylsulfate de sodium, le poloxamère, le poly(stéarate d'oxyéthylène) et les dérivés de polyoxyéthylène-huile de ricin ou des mélanges de ceux-ci.

6. Granulat selon l'une quelconque des revendications précédentes, contenant un plastifiant choisi dans le groupe des polymères de condensation d'oxyde d'éthylène et d'eau, des esters acides d'huile de sorbitol transformés avec de l'oxyde d'éthylène et leurs anhydrides, du citrate de triéthyle, du citrate d'acétyl-triéthyle, du citrate de tributyle, du propylèneglycol, du phtalate de diéthyle, de la triacétine, du citrate d'acétyl-tributyle et du sébacate de dibutyle ou des mélanges de ceux-ci.

7. Granulat selon l'une quelconque des revendications précédentes, contenant un agent d'ajustement du pH, un antimousse et/ou un lubrifiant.

8. Granulat selon l'une quelconque des revendications précédentes, contenant de l'eau ou un solvant ou des mélanges de ceux-ci, le solvant étant choisi dans le groupe formé par l'éthanol, l'alcool isopropylique et l'acétone.

9. Granulat selon l'une quelconque des revendications précédentes contenant
| | |
|---|---|
| 0,15 à 0,35% en poids | de Tamsulosine |
| 6 à 8% en poids | d'alginate de sodium |
| 55 à 65% en poids | de copolymère d'acide méthacrylique-acrylate d'éthyle 1:1 |
| 12 à 18% en poids | de béhénate de glycérol |
| 12 à 18% en poids | de maltodextrine |
| 0 à 10% en poids | d'adjuvants. |

10. Capsule de gélatine dure contenant le granulat selon l'une quelconque des revendications précédentes.

11. Procédé pour la préparation d'un granulat selon l'une quelconque des revendications 1 à 9, contenant de la Tamsulosine, dans lequel
- l'alginate de sodium, une première partie de la substance macromoléculaire et la substance hydrophobe sont mélangés, ce qui permet d'obtenir un mélange poudreux homogène,
- l'eau ou un solvant, une deuxième partie de la substance macromoléculaire et la Tamsulosine sont mélangés, ce qui permet d'obtenir un liquide de granulation homogène, et
- le liquide de granulation est ajouté, sous agitation intensive, au mélange poudreux homogène et le granulat obtenu est séché.

12. Procédé selon la revendication 11, dans lequel le mélange poudreux homogène est obtenu en ce que l'alginate de sodium, une première partie de la substance macromoléculaire, la substance hydrophobe et un liant sont mélangés.

13. Procédé selon la revendication 11, dans lequel le liquide de granulation est obtenu en ce que
- l'eau ou le solvant est chauffé, un plastifiant est ajouté et mélangé et la solution ainsi obtenue, contenant le plastifiant, est refroidie,
- la Tamsulosine et au moins un agent tensioactif sont mélangés et la deuxième partie de la substance macromoléculaire est ajoutée à ce mélange et on mélange, et
- le mélange ainsi obtenu est ajouté à la solution contenant le plastifiant.
